(19) Europäisches Patentamt
European Patent Office
Office européen des brevets

(11) Veröffentlichungsnummer: **0 073 302**
**B1**

(12) # EUROPÄISCHE PATENTSCHRIFT

(45) Veröffentlichungstag der Patentschrift: **22.05.85**

(51) Int. Cl.⁴: **C 07 D 473/08**

(21) Anmeldenummer: **82105005.1**

(22) Anmeldetag: **08.06.82**

(54) **Verfahren zur Herstellung von Dihydroxypropyltheophyllin.**

(30) Priorität: **25.08.81 DE 3133553**

(43) Veröffentlichungstag der Anmeldung: **09.03.83 Patentblatt 83/10**

(45) Bekanntmachung des Hinweises auf die Patenterteilung: **22.05.85 Patentblatt 85/21**

(84) Benannte Vertragsstaaten: **BE DE FR GB**

(56) Entgegenhaltungen:
**DE - B - 1 235 927**
**US - A - 2 575 344**

**CHEMICAL ABSTRACTS, Band 54, 1960, Spalten 1526h,1527, Columbus Ohio (USA);**
**JOURNAL OF THE CHEMICAL SOCIETY, 1956, Seite 3975, Letchworth (GB);**

**Die Akte enthält technische Angaben, die nach dem Eingang der Anmeldung eingereicht wurden und die nicht in dieser Patentschrift enthalten sind.**

(73) Patentinhaber: **Degussa Aktiengesellschaft, Weissfrauenstrasse 9, D-6000 Frankfurt am Main 1 (DE)**

(72) Erfinder: **Binder, Volker, Dr. Dipl.-Chem., Im Setzling 17, D-6460 Gelnhausen 1 (DE)**
Erfinder: **Merk, Wolfgang, Dr. Dipl.-Chem., Grünaustrasse 19, D-6450 Hanau 9 (DE)**
Erfinder: **Werle, Peter, Dr. Dipl.-Chem., Im Börner 43, D-6460 Gelnhausen 2 (DE)**

EP 0 073 302 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Theophyllin (1,3-Dimethylxanthin) und verwandte Naturstoffe werden pharmakologisch wegen ihrer Wirkungen auf das Zentralnervensystem und die Herz-Kreislauffunktion verwendet. Theophyllin hat eine ausgezeichnete Wirkung bei Status asthmaticus und Bronchialasthma. Die nur geringe Wasserlöslichkeit und dadurch bedingte langsame Resorption kann durch Überführung in geeignete wasserlösliche Derivate wesentlich verbessert werden.

Das Dihydroxypropyltheophyllin, 1,3-Dimethyl-7-(β, -dihydroxypropyl)-xanthin, stellt ein solches wasserlösliches Derivat dar. Seine Herstellung wird z.B. von P.V. Maney et al. in J. Am. Pharm. Assoc. 35, 266-72 (1946) durch Umsetzung von Theophyllin mit 3-Chlor-1,2-dihydroxypropan (Glycerin-α-monochlorhydrin) in Gegenwart äquivalenter Mengen Natronlauge beschrieben. Einige weitere Veröffentlichungen beschreiben ebenfalls die Reaktion von Theophyllin und Glycerin-α-monochlorhydrin in Gegenwart von KOH bzw. NaOH (F.F. Auslander Sci. Pharm. Proc. 25th 1965, 1, 75-7; US-PS 2 575 344; M. Samejima, Yakugarku Zasski 80, 1706 (1960); cf. CA 54 11648 g und CA 55 10439 i). D. Ishay empfiehlt die Umsetzung von Theophyllin mit KOH in Wasser mit anschliessendem Eindampfen zur Trockne. Anschliessend erfolgt die Zugabe von Glycerin-α-monochlorhydrin und Methanol. Ausbeute 78% vom Schmelzpunkt 155 bis 158°C (J. Chem. Soc. 3975, 1956).

Alle genannten Verfahren arbeiten in wässrigem Medium , mit nachfolgendem Eindampfen und Umkristallisation aus Methanol. Nachteilig ist vor allem der grosse Energiebedarf, der durch die hohe Verdampfungsenthalpie des Wassers bedingt wird, sowie der Anfall an Natriumchlorid (0,230 kg/kg Dihydroxypropyltheophyllin), das entsorgt und quantitativ vom Reaktionsprodukt abgetrennt werden muss.

Eine wesentliche Verbesserung des Verfahrens ergibt sich bei Ersatz des Glycerin-α-monochlorhydrins durch Glycid, da hier mit geringen Mengen katalytisch wirkender Substanzen gearbeitet werden kann, die Abtrennung grosser Mengen NaCl also entfällt. Ausserdem ist es möglich, die Reaktion in geeigneten Lösungsmitteln durchzuführen.

In Arch. Pharmaz. Ber. dt. pharmaz. Ges. 292/64, 234 (1939) berichtet H.J. Roth über die Umsetzung von Theophyllin und Theobromin mit 1,2-Epoxiden und setzt als Katalysatoren unter anderen Pyridinbasen ein. Das bevorzugte Lösungsmittel ist n-Propanol. Beim Nacharbeiten dieser Vorschrift in grösserem Massstab zeigt sich, dass durch das Pyridin eine starke Verfärbung der Reaktionslösung eintritt, die nur durch grosse Mengen an Aktivkohle beseitigt werden kann und weiterhin der Pyridingehalt sehr hoch ist (10%, bezogen auf eingesetztes Theophyllin). Dies führt zu erhöhtem apparativem Aufwand bei der Reinigung der Mutterlaugen.

Ausserdem ist das erhaltene Präparat selbst nach zweimaligem Umkristallisieren von ungenügender Reinheit (Roth gibt einen Schmelzpunkt von 154 bis 155°C an) und die Ausbeute nicht befriedigend , etwa 60% d. Th., bezogen auf Theophyllin.

Nach der deutschen Auslegeschrift 1 235 927 ist ein Verfahren zur Herstellung von β-Hydroxyalkyl-dimethylxanthinen bekannt, nach dem Theobromin oder Theophyllin mit 1,2-Epoxiden in Gegenwart von Aminoverbindungen umgesetzt wird.

Bei dem Versuch, dieses Verfahren auf die Herstellung von Dihydroxypropyltheophyllin unter Einsetzen von Glycid anzuwenden, entstand keine klare Lösung, d.h. das Theophyllin setzte sich nur zum Teil um.

Ausserdem war das entstandene Produkt nicht einheitlich und wies keinen definierten Schmelzpunkt auf.

Ziel der Erfindung war ein Verfahren zur Herstellung von Dihydroxypropyltheophyllin über Glycid und Theophyllin, bei dem des Reaktionsprodukt nicht nur in guter Ausbeute, sondern auch in hoher Reinheit anfällt.

Es wurde nun gefunden, dass sich diese Aufgabe lösen lässt, wenn man die an sich bekannte Umsetzung von Theophyllin mit Glycid in Gegenwart von Hydroxiden oder kurzkettigen Alkoholaten der Alkalimetalle oder Alkalisalzen der Pseudohalogenwasserstoffsäuren, die ein leicht polarisierbares Anion besitzen, durchführt.

Als Alkalimetalle kommen vor allem Lithium, Natrium und Kalium in Frage. Besonders bevorzugt ist Natrium. Von den Alkoholaten erweisen sich die Methylate oder Äthylate als besonders günstig.

Unter Pseudohalogenwasserstoffsäuren, die ein leicht polarisierbares Anion besitzen, werden verstanden: Cyanwasserstoff HCN; Rhodanwasserstoff HSCN, Cyansäure HOCN, Stickstoffwasserstoffsäure $HN_3$, Cyanamid $H_2N$-CN und Dicyanimid $HN(CN)_2$.

Besonders bevorzugt sind die Alkalihydroxide, vor allem Natriumhydroxid, ausserdem die Cyanate, vor allem Natriumcyanat oder Cyanamide wie z.B. Natriumcyanamid.

Die Mengen an einzusetzendem Katalysator liegen zwischen 0,01 bis 0,2 Mol pro Mol Theophyllin, vorzugsweise bei 0,05 bis 0,14 Mol pro Mol Theophyllin. Als Lösungsmittel für die Umsetzung kommen Wasser oder kurzkettige Alkohole wie Methanol, Äthanol oder Propanol in Frage. Letztere können auch als wasserhaltige Produkte mit einem Wassergehalt bis zu 20 Gew.-% eingesetzt werden. Besonders bevorzugt ist Methanol, da sich hierin Dihydroxypropyltheophyllin bei Siedehitze gut löst.

Theophyllin und Glycid werden in stöchiometrischen Mengen eingesetzt, bevorzugt mit einem geringen Überschuss an Glycid bis zu etwa 10 Mol-%.

Vorzugsweise wird in konzentrierten Lösungen gearbeitet. Als günstig erwiesen sich z.B. Konzentrationen der Einsatzstoffe von etwa 4 Mol pro Liter Lösungsmittel. Aber auch Molmengen bis zu 6,5 Mol ermöglichen ein einwandfreies Arbeiten. Sollte in gewissen Fällen das Absaugen des kristallisierten Produkts aus dem Reaktionsmedium schwierig sein, so lassen sich in an sich bekannter Weise durch Zusatz von Lösungsmitteln geringer Dichte wie den obengenannten Alkoholen in der Kälte ohne Ausbeuteverluste absaugbare Suspensionen herstellen.

Als günstig erwies sich, die Umsetzung zwischen Theophyllin und Glycid über einen längeren Zeitabschnitt, z. B. 3 bis 5 Stunden, hin vorzunehmen, da hierdurch die Gefahr der Nebenproduktbildung verringert wird.

Um die Konzentration an Glycid im Reaktionsmedium gering zu halten, wird dies am besten in die siedende Theophyllinsuspension eingetropft. Glycid und Theophyllin werden in handelsüblicher Qualität eingesetzt. Die Reaktionslösungen sind nur bei Anwendung von Alkalihydroxiden als Katalysatoren schwach gelblich gefärbt, sonst sind sie farblos.

Zur Gewinnung des Dihydroxypropyltheophyllins aus der Reaktionslösung gibt es verschiedene Möglichkeiten: entweder kann die Reaktionslösung auf tiefe Temperaturen von z.B. 0°C abgekühlt werden oder man kann — bei Verwendung von Alkoholen als Lösungsmittel — einen Teil dieses Lösungsmittels abdestillieren und die Kristallisation unter Rühren bei z.B. Raumtemperatur vornehmen.

Dihydroxypropyltheophyllin wird in Ausbeuten von 85 bis weit über 90%, bezogen auf eingesetztes Theophyllin, erhalten, siehe die Beispiele.

Die Reinheit des Reaktionsprodukts ist hoch; i.a. liegt der Anteil an nicht umgesetztem Theophyllin bei 0,1 bis 0,4 Gew.-%; wenn unbedingt nötig, kann sie durch eine übliche Umkristallisation, z.B. aus Methanol, noch weiter erhöht werden.

Wie die Beispiele zeigen, liegt der Schmelzpunkt des Dihydroxypropyltheophyllins zwischen 161 und 164°C, nach Umkristallisation bei genau 164°C. Hiermit entspricht das Produkt genau den Anforderungen der einschlägigen Pharmakopoen.

Das beim Einengen der Mutterlaugen noch anfallende restliche Dihydroxypropyltheophyllin ist durch Umkristallisieren ebenfalls in genügender Reinheit zu gewinnen.

Die Erfindung wird durch die folgenden Beispiele noch weiter erläutert:

*Beispiel 1*

108 g (0,6 Mol) Theophyllin und 2,4 g (0,06 Mol) NaOH werden in 320 ml Methanol aufgeschlämmt und unter Rückfluss zum Sieden erhitzt. Innerhalb von 5 h werden 49 g (0,66 Mol) Glycid zugetropft und nach beendeter Glycidzugabe die klare, schwach gelbe Lösung noch weitere 30 Minuten gerührt. Durch Abkühlen auf 0°C unter Rühren, Filtrieren und Waschen mit Methanol erhält man 142,6 g (92,6% d.Th., bezogen auf eingesetztes Theophyllin) Dihydroxypropyltheophyllin mit einem Schmelzpunkt von 162 bis 164°C.

*Beispiel 2*

108 g (0,6 Mol) Theophyllin werden mit 1,6 g (0,04 Mol) NaOH in 320 ml Methanol aufgeschlämmt und dann analog Beispiel 1 verfahren. Ausbeute 141,8 g 92,1% d.Th.), Schmelzpunkt 162 bis 163°C.

*Beispiel 3*

Das Beispiel wird analog Beispiel 1 ausgeführt. Als Katalysator werden 6,48 g (0,08 Mol) Kaliumcyanat eingesetzt.

Ausbeute 142,95 g (93,8% d.Th.), Schmelzpunkt 162°C.

*Beispiel 4*

Das Beispiel wird analog Beispiel 3 ausgeführt. Als Katalysator werden 5,1 g (0,08 Mol) Natriumcyanamid eingesetzt.

Ausbeute 135,6 g (89,2% d.Th.), Schmelzpunkt 161 bis 162°C.

Das so hergestellte Rohprodukt wird aus Methanol oder wässrigem Methanol umkristallisiert. Dazu werden 100 g in 250 ml Methanol (90%ig) zum Sieden erhitzt, filtriert und unter Rühren kristallisiert.

Ausbeute 89,5% d.Th., Schmelzpunkt 164°C.

*Beispiel 5*

108 g (0,6 Mol) Theophyllin werden mit 2,4 g (0,06 Mol) NaOH versetzt und mit 150 ml Wasser versetzt. Nach dem Erwärmen auf 90°C wird unter Rühren 49 g (0,66 Mol) Glycid innerhalb von 5 h zugetropft und noch ca. 30 Minuten nachreagieren gelassen.

Unter intensivem Rühren wird langsam abgekühlt, filtriert und der erhaltene Kristallbrei abgesaugt und mit Methanol gewaschen.

Ausbeute 129,6 g (85% d.Th.), Schmelzpunkt 163 bis 164°C.

**Patentansprüche**

1. Verfahren zur Herstellung von reinem Dihydroxypropyltheophyllin durch katalytische Umsetzung von Glycid und Theophyllin in Gegenwart von Lösungsmitteln, dadurch gekennzeichnet, dass man die Umsetzung in Gegenwart von Hydroxiden oder kurzkettigen Alkoholaten der Alkalimetalle oder Alkalisalzen der Pseudohalogenwasserstoffsäuren, die ein leicht polarisierbares Anion besitzen, durchführt.

2. Verfahren nach Anspruch 1, dadurch gekennzeichnet, dass man die Katalysatoren in Mengen von 0,01 bis 0,2 Mol Katalysator pro Mol Thophyllin einsetzt.

3. Verfahren nach Anspruch 2, dadurch gekennzeichnet, dass man zwischen 0,05 bis 0,14 Mol Katalysator pro Mol Theophyllin einsetzt.

4. Verfahren nach Anspruch 1 bis 3, dadurch gekennzeichnet, dass man als Katalysator Natriumhydroxid verwendet.

5. Verfahren nach Anspruch 1 bis 3, dadurch gekennzeichnet, dass man als Katalysator ein Alkalisalz von Cyanwasserstoff, Rhodanwasserstoff, der Cyansäure, der Stickstoffwasserstoffsäure, von Cyanamid oder Dicyanimid anwendet.

6. Verfahren nach Anspruch 1 bis 5, dadurch gekennzeichnet, dass man Natriumcyanat oder Natriumcyanamid einsetzt.

7. Verfahren nach Anspruch 1 bis 6, dadurch gekennzeichnet, dass man als Lösungsmittel für die Umsetzung Wasser oder Methanol verwendet.

**Claims**

1. A process for the production of pure dihydroxypropyl theophylline by catalytically reacting glycidol and theophylline in the presence of solvents, characterised in that the reaction is carried out in the presence of hydroxides or short-chain alcoholates of the alkali metals or alkali metal salts of pseudohydrohalic acids which contain an easily polarisable anion.

2. A process according to claim 1, characterised in that the catalysts are used in a quantity of from 0.01 to 0.02 mols of catalyst per mol of theophylline.

3. A process according to claim 2, characterised in that from 0.05 to 0.14 mols of catalyst are used per mol of theophylline.

4. A process according to claims 1 to 3, characterised in that sodium hydroxide is used as catalyst.

5. A process according to claims 1 to 3, characterised in that an alkali metal salt of hydrogen cyanide, hydrogen thiocyanate, cyanic acid, hydrazoic acid, cyanamide or dicyanamide is used as catalyst.

6. A process according to claims 1 to 5, characterised in that sodium cyanate or sodium cyanamide is used.

7. A process according to claims 1 to 6, characterised in that water or methanol is used as solvent for the reaction.

**Revendications**

1. Procédé pour la fabrication de dihydroxypropylthéophylline pure, par réaction calalytique de glycide et de théophylline, en présence de solvants, caractérisé en ce que l'on effectue la réaction en présence d'hydroxydes ou d'alcoolates à chaîne courte des métaux alcalins ou de sels alcalins des pseudohaloacides qui possèdent un anion facilement polarisable.

2. Procédé suivant la revendication 1, caractérisé en ce que l'on utilise les catalyseurs dans les proportions de 0,01 à 0,2 mole de catalyseur par mole de théophylline.

3. Procédé suivant la revendication 2, caractérisé en ce que l'on utilise de 0,05 à 0,14 mole de catalyseur par mole de théophylline.

4. Procédé suivant les revendications 1 à 3, caractérisé en ce que l'on utilise, comme catalyseur, l'hydroxyde de sodium.

5. Procédé suivant les revendications 1 à 3, caractérisé en ce que, comme catalyseur, on utilise un sel alcalin de l'acide cyanhydrique, de l'acide sulfocyanique, de l'acide cyanique, de l'acide hydroazoïque, de la cyanamide ou de dicyanimide.

6. Procédé suivant l'une des revendications 1 à 5, caractérisé en ce que l'on utilise le cyanate de sodium ou la cyanamide sodique.

7. Procédé suivant l'une des revendications 1 à 6, caractérisé en ce que comme solvant pour la réaction, on utilise l'eau ou le méthanol.